(19) **Europäisches Patentamt / European Patent Office / Office européen des brevets**

(11) **EP 3 701 992 B1**

(12) # EUROPEAN PATENT SPECIFICATION

(45) Date of publication and mention
of the grant of the patent:
**05.07.2023 Bulletin 2023/27**

(21) Application number: **19159694.9**

(22) Date of filing: **27.02.2019**

(51) International Patent Classification (IPC):
**A61M 16/20** *(2006.01)* **A61M 16/00** *(2006.01)*

(52) Cooperative Patent Classification (CPC):
**A61M 16/024; A61M 16/203;** A61M 16/0078;
A61M 16/101; A61M 16/208; A61M 2202/0283;
A61M 2205/18; A61M 2205/3331; A61M 2205/3334;
A61M 2205/3337; A61M 2205/505

(54) **GAS DELIVERY DEVICE WITH DEFORMABLE BAG AND DIFFERENTIAL PRESSURE SENSORS**

GASAUSGABEVORRICHTUNG MIT VERFORMBAREM BEUTEL UND DIFFERENZDRUCKSENSOREN

DISPOSITIF D'ADMINISTRATION DE GAZ COMPORTANT UN SAC DÉFORMABLE ET DES CAPTEURS DE PRESSION DIFFÉRENTIELLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**02.09.2020 Bulletin 2020/36**

(73) Proprietor: **L'AIR LIQUIDE, SOCIETE ANONYME POUR L'ETUDE ET L'EXPLOITATION DES PROCEDES GEORGES CLAUDE**
**75007 Paris (FR)**

(72) Inventor: **BOULANGER, Thierry**
**Newark, Delaware 19702 (US)**

(74) Representative: **Air Liquide**
**L'Air Liquide S.A.**
**Direction de la Propriété Intellectuelle**
**75, Quai d'Orsay**
**75321 Paris Cedex 07 (FR)**

(56) References cited:
WO-A1-2016/063168    WO-A1-2016/157104
US-A1- 2014 338 667    US-A1- 2017 232 221

**Description**

The present invention relates to a gas delivery device useable in various locations for treating patients, such as in hospitals, in physician or dentist offices, at home...

Some therapies require the administration of a gas mixture to patients. Thus, an equimolar (50%/50%) mixture of nitrous oxide ($N_2O$) and oxygen ($O_2$) can be used for relieving anxiety, providing light sedations or treating pain.

Generally speaking, a gas (i.e. gas or gas mixture) can be delivered to a patient either continuously or intermittently, i.e. periodically.

During a continuous administration, a continuous flow of gas exceeding the patient minute ventilation (i.e. the average volume of gas inhaled by the patient over 1 minute) is provided both during inhalation and exhalation phases of the patient. During inhalation phases, the patient inhales the gas contained into an inflatable reservoir, whereas during exhalation phases, the gas is stored into the inflatable reservoir to prepare the next inhalation phase.

Unfortunately, a continuous administration suffers several drawbacks:

- the minute ventilation of the patient must be frequently checked and the flow of gas adjusted, if necessary.
- depending on the gas flow, an important amount (> 33%) of gas is wasted, e.g. released to the atmosphere, without being inhaled by the patient.
- the reservoir is usually close to the patient, and further is cumbersome and noisy because of the turbulent delivery of the gas, leading to a discomfort for the patient.

Further, for ensuring an intermittent delivery of gas, an on-demand valve can be used, that opens proportionally only when a depression (negative pressure) occurs, e.g. during the inhalation phases of the patient, whereas it is closed during the exhalation phases of the patient.

However, on-demand valves are not ideal and also have drawbacks, such as :

- they require from the patients, some significant respiratory efforts for sustaining the respiratory demand, which can lead to an exhaustion of weak patients, such as those suffering from COPD.
- if the respiratory effort is not sufficient, the valve cannot be triggered and the gas is not delivered.
- in case of rapid shallow breathing, the response time of the valve can be too slow thereby negatively impacting the gas therapy.
- some mechanical parts (e.g. membranes, springs) of such valves lose their properties over time, involving some mismatch between the patient's demand and the gas delivery.

WO-A-2016/063168 teaches a system for controlling a leak flow rate during a respiratory therapy comprising a main gas passage in fluid communication with a pressure generator that can be a pump, a blower, a piston or bellows; a processing unit; one or several sensors for measuring parameters of the gas flow, such as an operating parameter of the pressure generator; and a leak valve for controlling leak during the therapy. The leak valve is arranged downstream of the main gas passage, namely close to the patient interface, i.e. respiratory mask or the like.

In this context, there is a need for an improved gas delivery system or device that provides comfortable and easy breathing to the patient and matches the patient's minute ventilation, including for weak patients, such as patients suffering from COPD or the like.

A solution according to the present invention concerns a gas delivery device comprising an inner gas passage in fluid communication with a deformable reservoir, and a processing unit, characterized it further comprises :

- a first differential pressure sensor cooperating with the processing unit for determining a pressure (P) in the deformable reservoir, i.e. a gas pressure,
- a proportional valve arranged on the inner gas passage for controlling the flowrate of gas in said inner gas passage, said proportional valve being arranged upstream of the deformable reservoir,

wherein the processing unit controls the proportional valve for adjusting the flowrate of gas passing through said proportional valve on the basis of said pressure (P) in the deformable reservoir.

Depending on the embodiment, the gas delivery device according to the present invention can comprise one or several of the following features :

- the first differential pressure sensor is configured or controlled for operating pressure measurements of pressure (P) at given time intervals, preferably every 20 msec or less, more preferably every 5 msec or less.
- the pressure (P) is determined at given time intervals, preferably every 20 msec or less, more preferably every 5 msec or less.

- the processing unit (51) is configured for processing at least one pressure measurement signal delivered by the first differential pressure sensor (281) and calculating the pressure (P) using at least one processed pressure measurement signal.
- the pressure (P) is calculated using signals delivered by the first differential pressure sensor and processed by the processing unit.
- the pressure (P) depends on the quantity of gas comprised into the deformable reservoir and is comprised between :

  • a pressure at rest (Prest) corresponding to a deformable reservoir full of gas, and
  • a given deflated pressure (Pmax) corresponding to a deformable reservoir at least partially deflated, with Pmax<Prest.

- the first differential pressure sensor is located immediately downstream of the deformable reservoir so as to measure the pressure (P) in the deformable reservoir.
- the first differential pressure sensor is arranged and configured for measuring the pressure (P) in the deformable reservoir.
- the processing unit is configured for controlling the proportional valve for setting or modifying (i.e. adjusting) the flowrate of gas traversing said proportional valve proportionally to the pressure (P). In other words, the flowrate of gas traversing the proportional valve is set by the processing unit so as to be proportional to the pressure (P).
- the processing unit controls the proportional valve for increasing or for decreasing the flowrate of gas traversing, i.e. passing through, the proportional valve based on the pressure (P). For instance, when the pressure (P) decreases, then the flowrate of gas is increased, as a pressure decrease means that the reservoir is at least partially deflated, and vice versa.
- the inner gas passage comprises one or several conduits or the like.
- the deformable reservoir is made of a flexible material, preferably a rubber material or the like, such as silicone rubber LSR from NuSil.
- the first differential pressure sensor is arranged in the inner gas passage, downstream of the deformable reservoir.
- the first pressure sensor is configured for sending a (i.e. one or several) pressure measurement signal at given time intervals, for instance every 20 msec or less, preferably every 5 msec.
- the processing unit is configured for:

  a) processing the pressure measurement signal(s) delivered by the first differential pressure sensor, and
  b) deducing from said pressure measurement signal(s), the pressure (P) in the deformable reservoir. Actually, the pressure (P) reflects a degree of inflation/deflation of the deformable reservoir, which corresponds to a residual volume of gas in the reservoir, for instance reservoir full, empty or in-between.

- the processing unit comprises a (or several) microprocessor(s), preferably a microcontroller.
- the processing unit comprises a (or several) microprocessor running one or several algorithms, preferably a (or several) microcontroller(s).
- the processing unit comprises a (or several) memory(ies) for storing information, data, signal measurements....., in particular look-up tables or the like.
- it further comprises a housing, preferably made of polymer or the like.
- the inner gas passage, the deformable reservoir, the proportional valve, the processing unit and the detection means are arranged in said housing.
- the inner gas passage is fluidly connected to a source of therapeutic gas.
- the source of therapeutic gas is a gas cylinder.
- the source of therapeutic gas contains $N_2O$, preferably a mixture of $N_2O$ and oxygen (O2).
- the source of therapeutic gas contains a binary mixture $N_2O/O_2$ containing 50 mol.% or less of $N_2O$ and oxygen ($O_2$) for the rest.
- the source of therapeutic gas contains an equimolar mixture $N_2O/O_2$ (50/50 mol.%).
- it further comprises an oxygen sensor is arranged in the inner gas passage for measuring the oxygen concentration in the gas flow circulating into said inner gas passage.
- the oxygen sensor is arranged upstream of the flexible reservoir.
- the inner gas passage is fluidly connected to an air entry line, preferably upstream of the flexible reservoir and/or downstream of the proportional valve, i.e. in-between.
- oxygen sensor is arranged between the air entry line and the flexible reservoir.
- it further comprises a flow sensor arranged in the inner gas passage for measuring the flow of gas (i.e. flowrate) circulating into the lumen of said inner gas passage.
- the flow sensor is arranged downstream of the proportional valve for measuring the flow of gas delivered by said proportional valve.

- the flow sensor is arranged upstream of the flexible reservoir, preferably upstream of the oxygen sensor, more preferably upstream of the air entry line.
- the flow sensor is a mass flow sensor or a differential pressure sensor.
- the deformable reservoir comprises (at rest) an internal volume of about between 0.5 and 3 L.
- the deformable reservoir comprises a peripheral wall having a thickness of between about 0.10 and 0.90 mm, typically of between about 0.25 and 0.50 mm.
- a (or several) one-way valve element(s) is arranged in the inner gas passage downstream of the deformable reservoir.
- the first differential pressure sensor is arranged between the deformable reservoir and the upstream fluid connection of the by-pass conduit to the inner gas passage.
- the first differential pressure sensor comprises a first sensing port at atmospheric pressure (i.e. ambient conditions) and a second sensing port arranged in the inner passage.
- it further comprises a (or several) one-way valve element arranged in the inner gas passage, downstream of deformable reservoir.
- the first differential pressure sensor is arranged between the deformable reservoir and the one-way valve element(s).
- it further comprises a second differential pressure sensor arranged so as to measure the pressure drop generated by said one-way valve, when a gaseous flow passes through it.
- the second differential pressure sensor is arranged in a by-pass conduct fluidly connected to the inner gas passage, at upstream and downstream locations of the one-way valve.
- the second differential pressure sensor delivers pressure signal(s) to the processing unit. Those signals are processed by the processing unit for deducing therefrom at least one pressure drop value corresponding to the pressure drop through the one-way valve element(s).
- it further comprises a power source for providing electric current to the different components or parts of the device in need thereof for working.
- it further comprises a man-machine interface, such as a touchscreen and buttons or the like.
- it further comprises a digital display.
- it further comprises an on/off actuator, such as a button, a touch switch or the like for switching on or off the device.
- it further comprises an alarm system for making the user aware in case of problem affecting the device or the gas, for instance a valve or sensor failure, a wrong gas composition (e.g. hypoxic mixture) ... The alarm system can provide audio and/or visual alert signals.

The present disclosure also concerns a method for providing a respiratory gas to a patient, i.e. a human being, in need thereof comprising :

a) providing a gas delivery device according to the present disclosure

b) delivering a respiratory gas to the patient's airways using said gas delivery device.

Depending on the embodiment, the method for providing a respiratory gas to a patient according to the present disclosure can comprise one or several of the following features :

- it further comprises providing a source of respiratory gas, preferably a gas cylinder containing the respiratory gas, especially a therapeutic gas.
- the respiratory gas contains a therapeutic gas containing one or several gaseous compounds, i.e. a gas or a gas mixture.
- the therapeutic gas contains $N_2O$.
- the therapeutic gas contains a mixture of $N_2O$ and oxygen ($O_2$).
- the therapeutic gas contains a mixture $N_2O/O_2$ containing 50 mol.% or less of $N_2O$ and oxygen ($O_2$) for the rest.
- the therapeutic gas contains an equimolar mixture $N_2O/O_2$ (50/50 mol.%).
- fluidly connecting the source of respiratory gas to the gas delivery device, preferably by means of a first flexible hose or the like.
- further fluidly connecting the gas delivery device to the patient's airways, preferably by means of a second flexible hose or the like.
- the respiratory gas is delivered to the patient by means of a respiratory interface, such as a respiratory mask or the like, preferably an oro-nasal mask.

Furthermore, the disclosure also concerns a gas delivery assembly comprising:

- a gas delivery device comprising an inner gas passage according to the present invention,
- a gas source, such as a gas cylinder equipped with a valve, in fluid communication with the inner gas passage of the gas delivery device for providing a respiratory gas to the gas delivery device, and
- a respiratory interface, such as a respiratory mask, in fluid communication with the inner gas passage of the gas delivery device for receiving the respiratory gas provided by said inner gas passage.

The present disclosure will be explained in more details in the following illustrative description of an embodiment of a gas delivery device according to the present disclosure which is made in references to the accompanying drawings among them :

- Fig. 1 is a schematic representation of an embodiment of a gas delivery device according to the present invention,
- Fig. 2 shows the internal architecture of the gas delivery device of Figure 1,
- Fig. 3 to 6 illustrate the first differential pressure sensor of the gas delivery device of Figure 2 for determining pressure P, and
- Fig. 7 to 9 represent pressures and volume curves obtained with the gas delivery device of Figures 1 and 2.

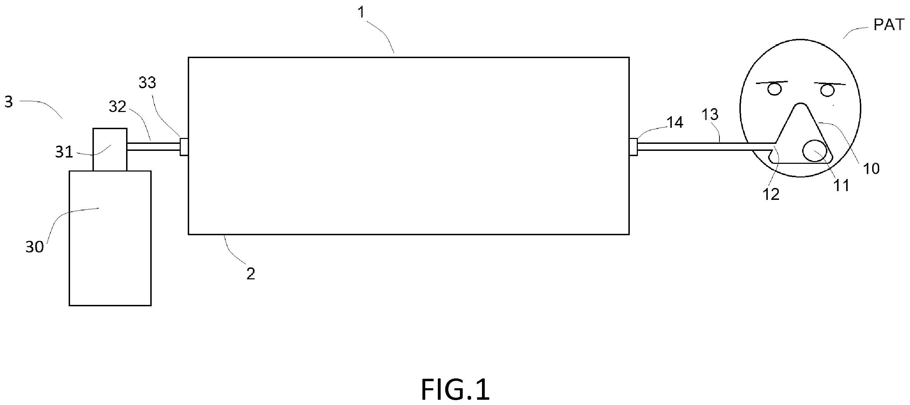

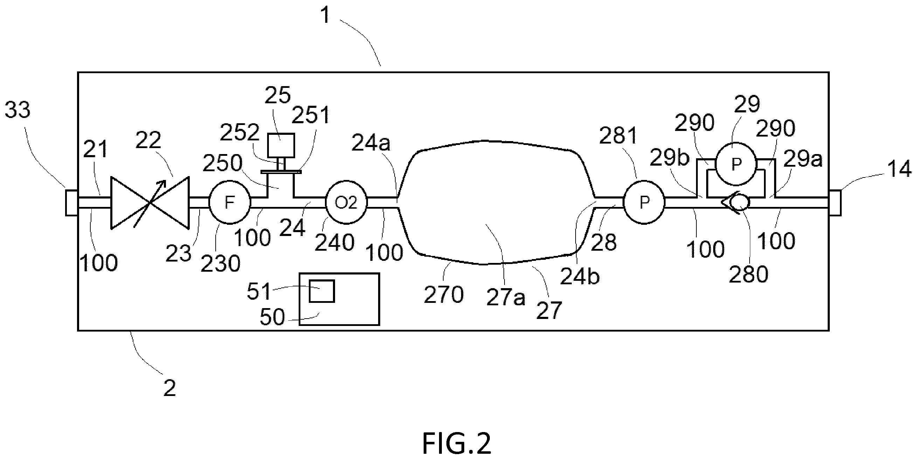

Figure 1 is schematic representation of an embodiment of a gas delivery device 1 according to the present invention. The gas delivery device 1 comprises a housing 2 or casing, for instance made of polymer, comprising components of the gas delivery device 1, as detailed below in reference to Figure 2.

A gas source 3, such as a gas cylinder 30 equipped with a valve 31, provides a respiratory gas, i.e. a gas or gas mixture, to the gas delivery device 1 by means of a gas line 32, such as a flexible hose or the like, that is fluidly connected to an inlet port 33 of the gas delivery device 1. The respiratory gas circulates into the gas delivery device 1, as detailed below, and is subsequently conveyed to a patient PAT by means of a flexible tube 13, i.e. a conduit, a hose or the like, that is fluidly connected to an outlet port 14 of the gas delivery device 1. The gas is administered to the patient PAT by means of a respiratory interface 10, such as a respiratory mask, that is fed by the flexible tube 13.

In Figure 1, the respiratory interface 10 is an oro-nasal mask covering the patient's mouth and nose. Other respiratory interfaces may also be suitable. The oro-nasal mask 10 exhibits an exhalation port 11 and inhalation port 12. The inhalation port 12 is fluidly connected to flexible tube 13 that conveys the gas to be inhaled from the outlet port 14 of the device 1 to the patient. The exhalation valve 11 is preferably a one-way valve that vents the $CO_2$-enriched gas exhaled by the patient P to the atmosphere, and that further prevents any backflow of ambient air coming from the atmosphere, when the patient PAT inhales respiratory gas, i.e. during inhalation phases. The one-way valve comprises a flexible silicone disk laying on a perforated surface that allows gas passing through unidirectionally, i.e. only in one way, for instance, the layout "membrane/perforated surface" of the valve sold by QOSINA under reference #97351.

The gas source 3 contains a pressurized gas, for instance an equimolar mixture (50%/50% ; mol.%) of $N_2O$ and $O_2$ at a maximal pressure of between 170 and 250 bars abs (when full of compressed gas). Valve 31 is preferably an integrated pressure-regulator valve 31 delivering the gas into hose 32 at a given reduced pressure, for instance a reduced pressure of 4 bar abs. Valve 31 is preferably protected by a rigid cap arranged around it (not shown).

In other words, Figure 1 shows a as delivery assembly 1, 3, 10 comprising the gas delivery device 1 of the present invention, a gas source 3, such as a gas cylinder 30 equipped with a valve 31, in fluid communication with the inner gas passage 100 of said gas delivery device 1 for providing a respiratory gas to the gas delivery device 1, preferably by means of a gas line 32, conduct or the like, and a respiratory interface 10, such as a respiratory mask, e.g. an oro-nasal mask, in fluid communication with the inner gas passage 100 of the gas delivery device 1 for receiving the respiratory gas provided by said inner gas passage 100, preferably by means of a flexible tube 13, hose or the like.

Figure 2 shows an embodiment of the different elements arranged into the housing 2 of the gas delivery device 1 according to the present invention, i.e. of the internal architecture of the gas delivery device 1 of Figure 1.

It comprises an electronic board 50 comprising a processing unit 51 including a (or several) microcontroller running an (or several) algorithm(s), which recovers and processes information, data and/or measurements provided by different actuators, sensors or the like.

An inner gas passage 100, i.e. a conduct or the like, is arranged in housing 2 between inlet port 33 and outlet port 14 so as to convey gas from inlet port 33 to outlet port 14. The inner gas passage 100 comprises several successive passage sections 21, 23, 24, 28.

The gas inlet port 33 carried by the rigid housing 2 of the gas delivery device 1 is in fluid communication with the upstream section 21 of inner gas passage 100. A proportional valve 22 is arranged on inner gas passage 100, preferably in the upstream part of inner gas passage 100 between first and second sections 21, 23. The proportional valve 22 is controlled by the microcontroller of the processing unit 51 for adjusting the gas flow circulating in the lumen of the inner gas passage 100 as detailed hereafter. Different types of proportional valves 22 can be used, such as proportional valves referenced IMI FAS FLATPROP or FESTO VEMR.

The gas flow passing through and exiting proportional valve 22 is recovered and conveyed by inner gas passage 100, namely the second section 23. A flow sensor 230 is arranged in inner gas passage 100 for measuring the flow (i.e. flowrate) of the gas provided by proportional valve 22.

Flow sensor 230 can be a mass flow sensor or a differential pressure sensor, preferably a differential pressure sensor. Flow sensor 230 is electrically connected to processing unit 51. Flow sensor 230 delivers a flow signal that is further processed by processing unit 51, namely the microcontroller. Preferably, a volumetric flow is obtained after conversion of the flow signal using a specific look-up table that is memorized in a memory cooperating with the processing unit 51.

Flow sensor 230 can also be used for detecting any default fault of proportional valve 22 or for determining the quantity of gas (i.e. volume) delivered by gas source 3.

Further, the gas delivery device 1 according to the present invention also comprises an air entry line 250, such as a conduit or the like, fluidly connected to the inner gas passage 100, downstream of the flow sensor 230, i.e. fluidly branched to third section 24. Air entry line 250 provides ambient air that mixes with the therapeutic gas traveling in the lumen of inner gas passage 100, preferably a $N_2O/O_2$ gas mixture.

An oxygen sensor 240 is further arranged in inner gas passage 100, downstream of the air entry line 250. Oxygen sensor 240 measures the oxygen concentration in the gas flow circulating into inner gas passage 100 after its mixing with air provided by air entry line 250, i.e. in third section 24. Oxygen sensor 240 has preferably a fast response time, for example 1s or less, preferably 200 msec or less. Paramagnetic sensors are useable, such as the sensor called Paracube Micro sold by Hummingbird Technologies.

Oxygen sensor 240 is also electrically connected to processing unit 51 and providing oxygen concentration measurements (i.e. signals) to processing unit 51.

The entering of air into air entry line 250 is controlled by a valve element 251, such as a disc shaped membrane, that normally prohibits air entering into air entry line 250. Valve element 251 cooperates with an actuator 25 comprising an acting part 252, like a stem or the like, mechanically coupled to the valve element 251. Actuator 25 is controlled by processing unit 51 and acts on the valve element 251, via acting part 252, for proportionally allowing or prohibiting the entering of air into air entry line 250. For instance, valve element 251 can be moved up for progressively allowing air entering into air entry line 250 by an air inlet (i.e. orifice or the like) or down for progressively prohibiting or stopping air entering into air entry line 250. Actuator 25 can be a linear actuator, for instance an actuator commercialized under reference 26DAM by Portescap.

The inner gas passage 100 of the gas delivery device 1 according to the present invention afterwards provides the gas flow to a deformable reservoir 27, in particular a flexible reservoir, arranged downstream of air entry line 250 and oxygen sensor 240, and in fluidic connection with inner gas passage 100, namely with third section 24.

Deformable reservoir 27 comprises a flexible peripheral wall 270 delimiting an internal volume 27a for the gas, thereby forming a "deformable bag" for the gas. At rest, deformable reservoir 27 exhibits an internal volume 27a of about between 0.5 and 3L for instance.

The flow of gas enters into the internal volume 27a of the deformable reservoir 27 through a reservoir inlet orifice 24a in fluid communication with inner passage 100.

Preferably, the properties of the deformable reservoir 27 are such that it is highly deformable. For instance, its peripheral wall 270 has a thickness of between about 0.25 and 0.5 mm and is made of a flexible, biocompatible silicone rubber, such as LSR series commercialized by NuSil.

The gas exits the internal volume 27a of reservoir 27 by a reservoir outlet orifice 24b that is fluidly connected to a downstream section 28 of inner gas passage 100 that terminates at outlet port 14. A first differential pressure sensor 281 configured to measure negative pressures (i.e. compared to atmospheric pressure) down to -5mb, is preferably arranged in downstream section 28.

In other embodiments, the first differential pressure sensor 281 can be arranged upstream of reservoir 27 or in reservoir 27.

The first differential pressure sensor 281 comprises two sensing ports including a first sensing port kept at atmospheric conditions (i.e. atmospheric pressure) and a second port sensing arranged in downstream section 28 of inner passage 100. Downstream section 28 is large enough to not oppose any resistance upon flow progression. Consequently, the pressure existing where the first differential pressure sensor 281 is located, is considered equivalent to the pressure P in reservoir 27.

For instance, the differential pressure sensor called "SDP3X series" from Sensirion can be used.

At frequent time intervals, for instance every 5 msec, said first differential pressure sensor 281 sends a pressure measurement signal to the processing unit 51 which processes said pressure measurement signal for determining, via a specific lookup table, the pressure P in said reservoir 27.

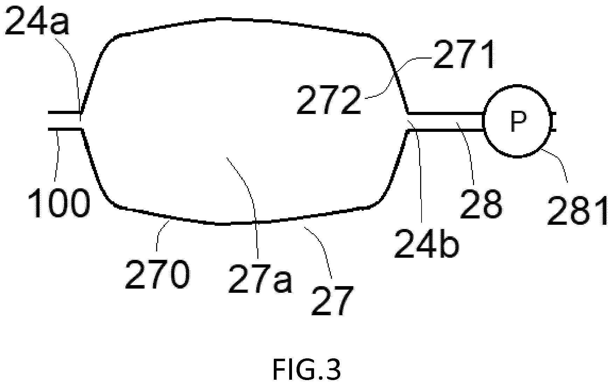

Figures 3-6 show the flexible reservoir 27 of the gas delivery device of Figure 2 in different inflation/deflation states, and illustrate the relationship between pressure P and said inflation/deflation states.

Figure 3 shows the reservoir 27 at rest, e.g. ambient condition into internal volume 27a which is full of gas. In this state, microcontroller 51 controls the first differential pressure sensor 281 to perform a pressure measurement. As above explained, microcontroller 51 determines, via a look-up table or the like, the pressure P existing in reservoir 27. This pressure is called $P_{REST}$ or pressure 'at rest' which is equivalent to 0 as the pressure in reservoir 27 equals atmospheric pressure (i.e. 1 atm).

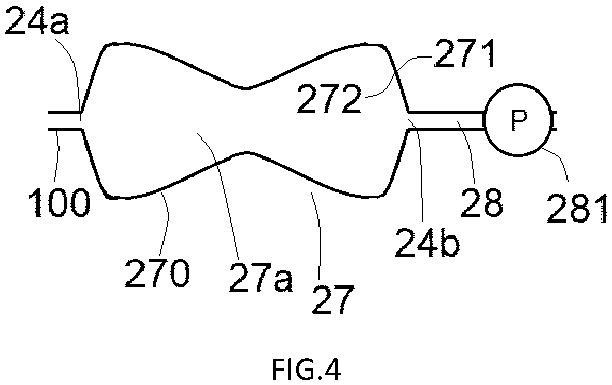

The transition between Figure 3 and Figure 4 illustrates a deflation of reservoir 27, which occurs when the force acting on the outside part 271 (i.e. its outer surface) of peripheral wall 270 is greater than the sum of the force opposed by said peripheral wall 270 (i.e. its "flexibility") and the force acting on the inside part 272 of said peripheral wall 270.

Figure 4 represents a state of partial deflation at equilibrium, e.g. when the sum of said forces acting on reservoir 27 equals to about 0. In this state of partial deflation, the force opposed by peripheral wall 270 is still negligible and it can be determined, for example, that the equilibrium is reached when the pressure in internal volume 27a, which is proportional to the force acting on inside part 272 of peripheral wall 270, is about 0.2 mbar smaller than ambient pressure, i.e. -0.2 mbar. The microcontroller 51 controls the first differential pressure sensor 281 to perform a pressure measurement for measuring a pressure P in reservoir 27 of about -0.2 mbar.

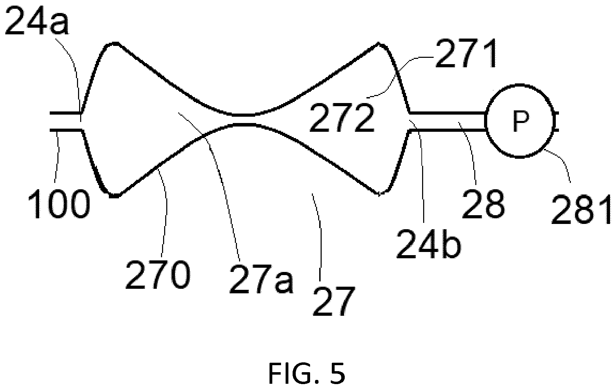

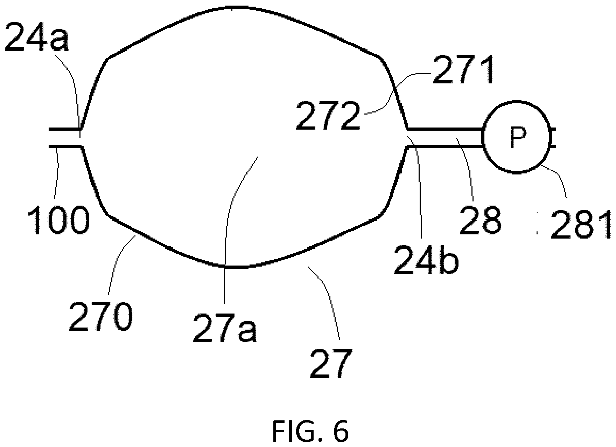

Figures 5 and 6 show other states of reservoir 27.

In Figure 5, reservoir 27 is further deflated compared to Figures 3 and 4. The more reservoir 27 is deflated, the more the force opposed by its peripheral wall 270 increases until becoming predominant. Consequently, the negative pressure in internal reservoir 27a may quickly drop, especially in a nonlinear way (e.g. the relationship between a degree of deflation of reservoir 27 and resulting pressure P in said reservoir 27 is nonlinear).

In Figure 5, for example, although reservoir 27 is slightly more deflated than in Figure 4, the pressure in internal volume 27a has dramatically decreased, to reach about -2 mbar (as opposed to -0.2 mbar of Fig. 4). Again, the microcontroller 51 controls the first differential pressure sensor 281 to perform a measurement for measuring said pressure of -2 mbar.

Assuming that the pressure P measured in Figure 4 represents a deflation above which the force opposed by the peripheral wall 270 of reservoir 27 quickly becomes non-negligible, said pressure P represents a threshold that is called "$P_{MAX}$".

Both pressures $P_{REST}$ and $P_{MAX}$ can be factory calibrated and stored in the memory by microcontroller 51 as pressure thresholds, i.e. upper and lower boundaries, whose role will be explained hereafter.

Alternately, in Figure 6, reservoir 27 is over inflated, i.e. the pressure existing in internal volume 27a of reservoir 27 is therefore greater than ambient pressure (i.e. > 1 atm). In other words, the pressure P in reservoir 27 measured by first differential pressure sensor 281 and processed by processing unit 51 is positive, e.g. greater than 0 bar (i.e. > 0 bar).

As discussed, the gas exits the internal volume 27a of reservoir 27 by a reservoir outlet orifice 24b that is fluidly connected to a downstream section 28 of inner gas passage 100 that terminates at outlet port 14.

A (or several) one-way valve element 280 is arranged in the inner gas passage 100, downstream of reservoir 27, namely between reservoir outlet orifice 24b and outlet port 14 of housing 2, for preventing any backflow of gas. Thus, gas exhaled by patient PAT are vented only through exhalation port 11 of mask 10 and cannot return into reservoir 27. One-way valve 280 is preferably designed such that a very low pressure drop (i.e. < 0.2 mbar) is generated across it, when a flow of gas travels through it. In another embodiment, several one-way valve elements 280 can also be used in lieu of only one, for example 3 to 5 arranged in parallel (not shown).

It is further provided a second differential pressure sensor 29 for measuring the pressure drop generated by said one-way valve 280 when a flow is passing through it. second differential pressure sensor 29 is arranged on a by-pass conduit 290 fluidly connected to the inner gas passage 100, upstream and downstream ('U'-shape) of said one-way valve 280 for allowing a measurement of the pressures in inner gas passage 100, at two locations 29a, 29b, namely upstream 29b and downstream 29a of one-way valve 280. Pressure signals measured by the second differential pressure sensor 29 are sent and then processed by the microcontroller of the processing unit 51. Typically, pressure signals are converted into a flow using a specific look-up table corresponding to the pressure-flow relationship of one-way valve 280. For instance, the differential pressure sensor "SDP3X series" from Sensirion can be used.

A power source (not shown) is preferably arranged in housing 2, such as a rechargeable battery, for delivering electric current (i.e. power) to all the components working with electric current, such as sensors, processing unit, controlled-valves, first differential pressure sensor, man-machine interface, digital display....

The gas delivery device 1 according to the present invention works as follows during therapy initiation, therapy administration and at the end of therapy.

Therapy initiation

Therapy initiation corresponds to the phase, when the device 1 is switched on and the patient PAT equipped with an oro-nasal mask 10 and starts to breath respiratory gas.

By controlling the linear actuator 25 via the microcontroller of the processing unit 51, membrane 251 is pulled from the air entry conduit 250, liberating an inlet orifice for ambient air to enter into air entry conduit 250 (cf. Fig. 2). At this stage, the microcontroller commands proportional valve 22 to remain closed so that the only gas travelling into inner gas passage 100 is ambient air. The deformable reservoir 27, that is in fluid communication with air entry conduit 250, is also at ambient conditions and in its "rest" position, e.g. no constraint or force applies to it, and its internal volume 27a is maximal. In this state, pressure P that is measured, corresponds to $P_{REST}$ as shown in Fig. 3.

When the patient starts to inhale, as exhalation valve 11 is closed, a slight depression occurs at the inhalation port 12 of mask 10, which spreads into tubing 13, outlet 14 and downstream section 28 of inner gas passage 100. As the gas pressure into the internal volume 27a of reservoir 27 equals to atmospheric pressure, a positive differential pressure exists across one-way valve 280 that allows some gas passing through said one-way valve 280 to supply patient's respiratory demand. Consequently, the internal volume 27a of reservoir 27 depletes and the reservoir 27 collapses accordingly, creating in turn a slight depression into internal volume 27a which draws ambient air into air entry conduit 250. The deformation of flexible reservoir 27 depends on the instantaneous demand of the patient PAT and ability of the inlet orifice to let ambient air being drawn into air entry conduit 250, and afterwards reservoir 27. The deformable reservoir 27 progressively deflates/collapses and the pressure P decreases to sub-atmospheric pressures, as shown in Figures 4 and 5. In Figure 5, the deformable reservoir 27 is over-deflated as explained above.

When the patient starts to exhale into mask 10, exhalation valve 11 of mask 10 opens to vent the exhaled $CO_2$-enriched gas, which creates a small positive pressure in mask 10, which spreads from inhalation port 12 to the downstream section of inner gas passage 100, via tubing 13 and outlet 14. As the internal volume 27a of reservoir 27 is at atmospheric pressure or at a slightly negative pressure (as in Figure 4), a negative differential pressure exists across one-way valve 280 that forces said one-way valve 280 to close. While patient is exhaling, the propensity of reservoir 27 to get back to its resting state, thanks to its flexibility, continues to draw ambient air, if necessary, by virtue of the fluid connection existing between air entry conduit 250, inner gas passage 100 and said reservoir 27. As a result, the reservoir 27 goes back to its resting position (i.e. with P = $P_{REST}$), e.g. internal volume 27a is maximal and the patient is ending the exhalation phase. The inhalation/exhalation sequences can then start again.

During initiation phase, a calibration of oxygen sensor 240 can be operated as the gas travelling into passage 100 is ambient air (i.e. 21% $O_2$). Once, the oxygen sensor 240 is stabilized, e.g. has been in contact with ambient air for enough time, the processing unit 51 can perform a calibration of said oxygen sensor 240. This calibration point helps determining a new look-up table that takes into account any drift having occurred in said oxygen sensor 240 to guarantee an appropriate accuracy of the oxygen concentration measurement.

Therapy administration

The processing unit 51 commands the linear actuator 25 to push the membrane 251 back to a close position thereby occluding the air entry conduit 250 and preventing any air ingress. The only gas circulating into inner passage 100 is delivered by proportional valve 22, for instance an $O_2/N_2O$ mixture (50/50 mol%). The therapy then can start and the patient PAT can inhale and exhale gas thanks to oro-nasal mask 10.

As the exhalation valve 11 is closed and the pressure into the internal volume 27a of reservoir 27 equals atmospheric pressure, the slight depression that occurs at the inhalation port 12 of mask 10 allows gas passing through one-way valve 280 to supply the patient's respiratory demand. Consequently, the internal volume 27a of reservoir 27 depletes and the reservoir 27 collapses (i.e. is deformed) accordingly.

While the reservoir 27 depletes, the pressure P inside reservoir 27 decreases accordingly. Using first differential pressure sensor 281 at regular intervals, such as every 5 msec, processing unit 51 can determine the evolution of the pressure P in said reservoir 27. The microcontroller 51 is configured to ensure that, at any time, the pressure P in reservoir 27 is as close as possible of $P_{REST}$, but never greater than it and never less than $P_{MAX}$.

Indeed, these two upper and lower thresholds, i.e. $P_{REST}$ and $P_{MAX}$, define an authorized range of deflation for reservoir 27 during inhalation phases of the patient.

More precisely, if the pressure P measured by microcontroller 51 :

- is greater than $P_{REST}$ corresponding to the position at rest of reservoir 27, then the reservoir 27 is over-inflated as shown in Figure 6. In other words, the pressure in internal volume 27a of reservoir 27 exceeds the atmospheric pressure (i.e. > 1 atm), meaning that some gas is forced out of reservoir 27 and wasted to ambient.
- becomes less than $P_{MAX}$, then the level of deflation of reservoir 27 is too important, as shown in Figure 5. This forces the patient PAT to generate important negative pressures to sustain its respiratory demand, e.g. overcoming the negative pressure existing in internal volume 27a, causing a discomfort for the patient.

In other words, over the course of the inhalation, microcontroller of processing means 51 ensures that the reservoir 27 that is at "rest" (but not overinflated), while providing mechanisms not to exceed a partial deflation above which the patient PAT might feel a discomfort breathing in said reservoir 27.

In other words, the device 1 of the present invention is configured for operating in a comfort zone for the patient, corresponding to the range defined by pressures $P_{REST}$ and $P_{MAX}$.

For doing so, the microcontroller of the processing unit 51 controls proportional valve 22 that is fed with therapeutic gas by gas source 3, for allowing a passage of therapeutic gas (e.g $N_2O/O_2$), through said proportional valve 22, at a flowrate which related (e.g. proportional) to the pressure P measured as explained below.

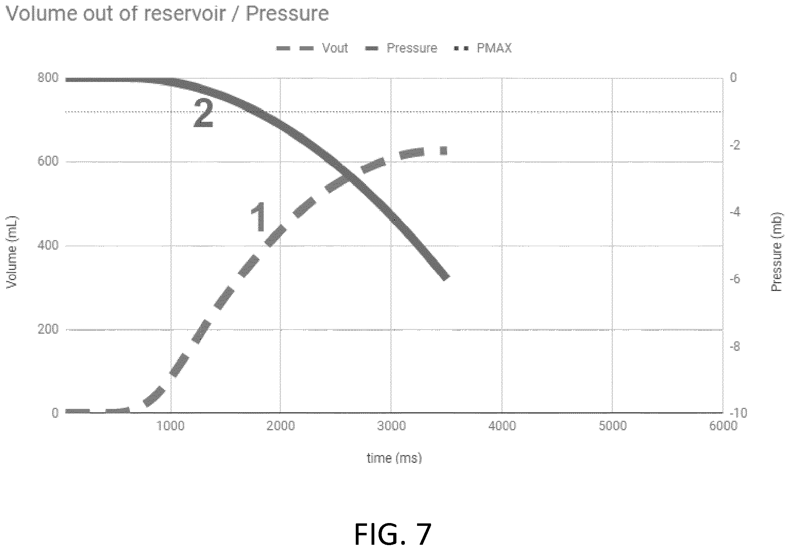

Figures 7 to 9 represent pressure and volume curves that can be obtained with a gas delivery device 1 as shown in Figures 1 and 2 equipped with a deformable reservoir 27 of for instance 1 L (at rest).

In Figures 7-9, the position at rest of said reservoir 27 is represented by a pressure P of 0 mbar (i.e. $P_{REST}$ = 0 mbar), whereas the over-deflated state (cf. Figure 5), is represented by a negative pressure of -10mbar. A desired pressure $P_{MAX}$ is here set to about -1mbar, which corresponds to about 400 mL of gas. The "comfort zone" of the deformable reservoir 27 is hence of between 0 (i.e. $P_{REST}$) and -1mbar (i.e. $P_{MAX}$).

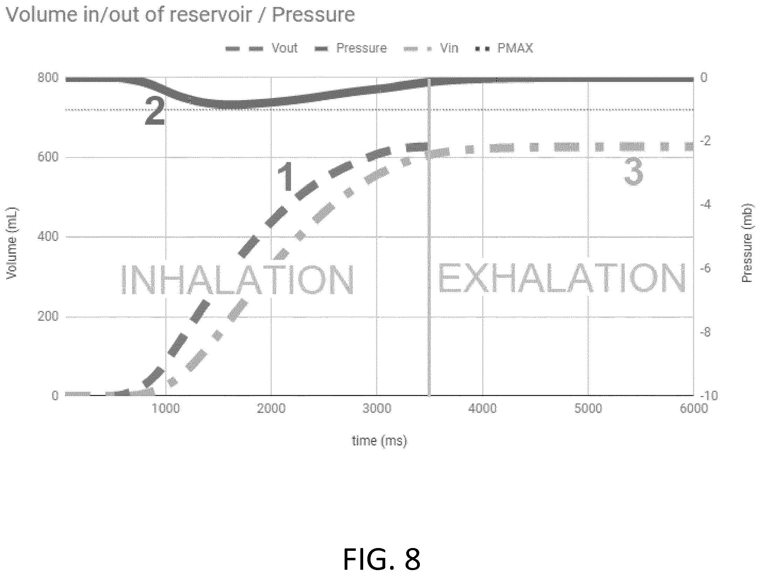

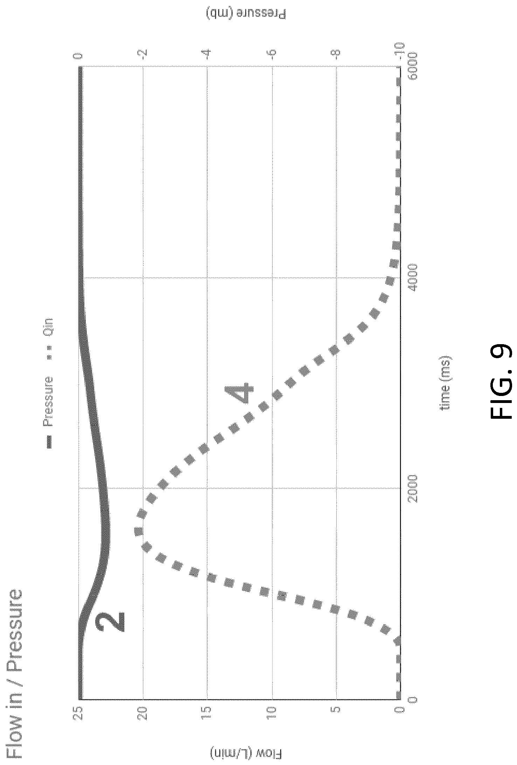

In Figure 7, the proportional valve 22 stays closed, whereas in Figures 8 and 9, proportional valve 22 delivers a flow that ultimately replenish the deformable reservoir 27.

More precisely, in Figure 7, when the microcontroller of the processing unit 51 controls proportional valve 22 to stay closed so that no therapeutic mixture enters into reservoir 27, a volume "Vout" (i.e. curve 1 : "- - - -" in Fig. 7) of gas is drawn out of the reservoir by a patient PAT over the course of an inhalation, which corresponds to a deflation represented by the "pressure" curve (i.e. curve 2 : "-----" in Fig. 7). At the end of the inhalation, the reservoir has been emptied by about 650 mL and deformed consequently, i.e. P is of about -6mbar, which exceeds the set threshold $D_{MAX}$ of - 1mbar. This means that about 50% of the inhalation time occurs outside the comfort zone.

Further, in Figure 8, the microcontroller of the processing unit 51 controls proportional valve 22 so that the flow of gas delivered by said proportional valve 22 is proportional to the pressure P (i.e. compared to $P_{REST}$) determined by said microcontroller and first differential pressure sensor 281 as, for instance, given by the following formula :

$$\text{Gas Flow (L/min)} = Q_{MAX}.[(P-P_{REST})/(P_{MAX}-P_{REST})]$$

In other words, if $P=P_{REST}$, then the flow delivered by proportional valve 22 is equal to 0 Umin, whereas if $P=P_{MAX}$, proportional valve 22 is piloted to remain fully opened for delivering a maximum gas flow (called $Q_{MAX}$), for example $Q_{MAX}$ = 40 L/min. Of course, the gas flow is proportional in-between, i.e. proportional valve 22 is controlled to be partially opened.

In the example of Figure 8, the volume of gas "Vout" (curve 1 : "- - - -") drawn by patient PAT is partially compensated by an incoming volume "Vin" (curve 3 : "- . - . - ") which opposes the deflation of reservoir 27. The buildup of such volume "Vin" (curve 3) in reservoir 27 is made possible by the incoming flow "Qin" (curve 4 : " .... ") as shown in Figure 9, delivered by proportional valve 22 following for instance an algorithm implemented by the microcontroller of the processing unit 51.

If the maximum deflation (that occurred in Figure 7) exceeds the comfort zone of reservoir 27, the "pressure" (curve 2 : "---" in Fig. 8 and Fig. 9), over the course of the inhalation, is down to less than -0.5 mbar, i.e. well within the expectations. In this case, at the end of inhalation, the "pressure" (curve 2) remains slightly below $P_{REST}$, e.g. at about -0.1 mbar.

When the patient PAT exhales, the exhalation valve 11 of mask 10 opens to vent the exhaled $CO_2$-enriched gas, creating a slight positive pressure into mask 10, thereby closing one-way valve 280. Following the rule deployed during the inhalation phase, the microcontroller controls the proportional valve 22 to ensure that the reservoir 27 is back to or near its position at rest, corresponding to a pressure P measured by first differential pressure sensor 281 equal or close to $P_{REST}$ as shown in Figures 8 and 9 where the "pressure" (i.e. curve 2 : "---") slowly goes back to 0mb, namely the position at rest of the reservoir 27.

Of course, different mechanisms can be provided for ensuring that reservoir 27 stays in the comfort zone/range, such as intrinsic properties of reservoir 27 (e.g. determination of $P_{MAX}$, internal volume 27a...), technical features of proportional valve 22 (e.g. maximum flow $Q_{MAX}$...), sophistication the algorithm deployed by microcontroller (e.g. utilization of Proportional Integral Derivative control...)...

At this stage, the patient PAT is transitioning to a new inhalation phase and the device 1 is ready to supply the upcoming gas demand as the reservoir 27 is fully inflated, i.e. full of therapeutic gas.

<u>End of therapy</u>

The end of the therapy is determined by a time limit or by the control of the user for example. The stepper motor pulls the membrane 251 to create a passage 251a for ambient air that can enter into air entry conduit 250, whereas the therapeutic gas supply is stopped by closing proportional valve 22. The patient can then quietly recover from any lightheaded sensation that frequently occurs during $N_2O$ administration as ambient air progressively replace the therapeutic gaseous mixture in reservoir 27.

In other words, the reservoir 27 contains 50% (%mol) of $N_2O$ or less.

In particular cases, it may be wise to dilute the therapeutic mixture with ambient air, e.g. air provided by the air entry conduit 250.

Thus, it can be determined that having a $N_2O$ concentration $C_{N2O}$ results in a specific O2 concentration $C_{O2}$:
$C_{O2} = 21 + 29. C_{N2O}/50$

For instance, a set concentration $C_{N2O}$ of 40% yields to a resulting concentration $C_{O2}$ of 44%.

Microcontroller of processing means 51 controls both proportional valve 22 and linear actuator 25 so that they cooperate together.

A first step consists in fixing the membrane 251 at a given position that creates a passageway 251a and allows ambient air to enter into air entry conduit 250. The position of membrane 251 depends on the desired $N_2O$ concentration $C_{N2O}$ with respect to the concentration of $N_2O$ in the therapeutic mixture (e.g. 50%).

The determination of the position of membrane 251 can be made by the microcontroller thanks to a specific lookup table providing a correlation between set $N_2O$ concentration and membrane 251 position. Once the position of membrane 251 is determined, the microcontroller controls the proportional valve 22 to provide the adequate amount of therapeutic mixture. This can be done by performing a closed-loop regulation on oxygen sensor 240 with a low response time, e.g. about 200ms or less.

The control of the proportional valve 22 is set and actualized in real time by the microcontroller to keep the oxygen concentration $C_{O2}$ in inner gas passage 100 at the desired value, e.g. 44%.

Generally speaking, a gas delivery device 1 according to the present invention can be used for providing a respiratory gas, especially a therapeutic gas, preferably containing $N_2O$ and oxygen, to a patient in need thereof.

**Claims**

1. Gas delivery device (1) comprising an inner gas passage (100) in fluid communication with a deformable reservoir (27), and a processing unit (51), characterized it further comprises :

   - a first differential pressure sensor (281) cooperating with the processing unit (51) for determining a pressure (P) in the deformable reservoir (27), and
   - a proportional valve (22) arranged on the inner gas passage (100) for controlling the flowrate of gas in said inner gas passage (100), said proportional valve (22) being arranged upstream of the deformable reservoir (27),

   wherein the processing unit (51) controls the proportional valve (22) for adjusting the flowrate of gas passing through said proportional valve (22) on the basis of said pressure (P) in the deformable reservoir (27).

2. Gas delivery device according to Claim 1, **characterized in that** the the first differential pressure sensor (281) is configured or controlled for operating pressure measurements of pressure (P) at given time intervals, preferably every 20 msec or less, more preferably every 5 msec or less.

3. Gas delivery device according to any one of the preceding Claims, **characterized in that** the processing unit (51) is configured for processing at least one pressure measurement signal delivered by the first differential pressure sensor (281) and calculating the pressure (P) using at least one processed pressure measurement signal.

4. Gas delivery device according to any one of the preceding Claims, **characterized in that** the processing unit (51) is configured for controlling the proportional valve (22) for setting or modifying the flowrate of gas traversing said proportional valve (22), proportionally to the pressure (P).

5. Gas delivery device according to any one of the preceding Claims, **characterized in that** the first differential pressure sensor (281) is arranged in the vicinity of the deformable reservoir (27).

6. Gas delivery device according to any one of the preceding Claims, **characterized in that** the processing unit (51) controls the proportional valve (22) for increasing or for decreasing the flowrate of gas traversing the proportional valve (22) based on the pressure (P).

7. Gas delivery device according to any one of the preceding Claims, **characterized in that** the first differential pressure sensor (281) is arranged in the inner gas passage (100) downstream of the deformable reservoir (27).

8. Gas delivery device according to any one of the preceding Claims, **characterized in that** the processing unit (51) comprises a microprocessor, preferably a microcontroller.

9. Gas delivery device according to any one of the preceding Claims, **characterized in that** the first differential pressure sensor (281) comprises a first sensing port at atmospheric pressure and a second sensing port arranged in the inner passage 100.

10. Gas delivery device according to any one of the preceding Claims, **characterized in that** it further comprises a (or several) one-way valve element (280) arranged in the inner gas passage (100) downstream of deformable reservoir (27).

11. Gas delivery device according to any one of the preceding Claims, **characterized in that** the first differential pressure sensor (281) is arranged between the deformable reservoir (27) and the one-way valve element (280).

12. Gas delivery device according to any one of the preceding Claims, **characterized in that** it further comprises a second differential pressure sensor (29) arranged so as to measure the pressure drop generated by said one-way valve (280).

13. Gas delivery device according to any one of the preceding Claims, **characterized in that** the second differential pressure sensor (29) is arranged in a by-pass conduct (290) fluidly connected to the inner gas passage (100), at upstream and downstream locations (29a, 29b) of the one-way valve (280).

14. Gas delivery device according to any one of the preceding Claims, **characterized in that** the second differential pressure sensor (29) delivers pressure signals to the processing unit (51).

15. Gas delivery assembly (1, 3, 10) comprising:

- a gas delivery device (1) comprising an inner gas passage (100) according to any one of the preceding Claims,
- a gas source (3), such as a gas cylinder (30) equipped with a valve (31), in fluid communication with the inner gas passage (100) of the gas delivery device (1) for providing a respiratory gas to the gas delivery device (1), and
- a respiratory interface (10), such as a respiratory mask, in fluid communication with the inner gas passage (100) of the gas delivery device (1) for receiving the respiratory gas provided by said inner gas passage (100).

**Patentansprüche**

1. Gasabgabevorrichtung (1), umfassend einen inneren Gasdurchgang (100) in Fluidkommunikation mit einem verformbaren Reservoir (27) und eine Verarbeitungseinheit (51), **dadurch gekennzeichnet, dass** sie des Weiteren umfasst:

- einen ersten Differenzdrucksensor (281), der mit der Verarbeitungseinheit (51) zusammenarbeitet, um einen Druck (P) in dem verformbaren Reservoir (27) zu bestimmen, und
- ein Proportionalventil (22), das an dem inneren Gasdurchgang (100) angeordnet ist, um die Flussrate von Gas in dem inneren Gasdurchgang (100) zu steuern, wobei das Proportionalventil (22) stromaufwärts von dem verformbaren Reservoir (27) angeordnet ist,

wobei die Verarbeitungseinheit (51) das Proportionalventil (22) steuert, um die Flussrate von Gas, welches das Proportionalventil (22) passiert, basierend auf dem Druck (P) in dem verformbaren Reservoir (27) anzupassen.

2. Gasabgabevorrichtung nach Anspruch 1, **dadurch gekennzeichnet, dass** der erste Differenzdrucksensor (281) ausgestaltet oder gesteuert ist, um Druckmessungen des Drucks (P) in gegebenen Zeitintervallen auszuführen, vorzugsweise alle 20 ms oder weniger, bevorzugter alle 5 ms oder weniger.

3. Gasabgabevorrichtung nach einem der vorhergehenden Ansprüche, **dadurch gekennzeichnet, dass** die Verarbeitungseinheit (51) ausgestaltet ist, um mindestens ein von dem ersten Differenzdrucksensor (281) abgegebenes Druckmessungssignal zu verarbeiten und den Druck (P) unter Verwendung von mindestens einem verarbeiteten Druckmessungssignal zu berechnen.

4. Gasabgabevorrichtung nach einem der vorhergehenden Ansprüche, **dadurch gekennzeichnet, dass** die Verarbeitungseinheit (51) ausgestaltet ist, um das Proportionalventil (22) so zu steuern, dass die Flussrate von Gas, welches das Proportionalventil (22) durchquert, proportional zu dem Druck (P) eingestellt oder modifiziert wird.

5. Gasabgabevorrichtung nach einem der vorhergehenden Ansprüche, **dadurch gekennzeichnet, dass** der erste Differenzdrucksensor (281) in der Nähe des verformbaren Reservoirs (27) angeordnet ist.

6. Gasabgabevorrichtung nach einem der vorhergehenden Ansprüche, **dadurch gekennzeichnet, dass** die Verarbeitungseinheit (51) das Proportionalventil (22) steuert, um die Flussrate von Gas, welches das Proportionalventil (22) durchquert, basierend auf dem Druck (P) zu erhöhen oder abzusenken.

7. Gasabgabevorrichtung nach einem der vorhergehenden Ansprüche, **dadurch gekennzeichnet, dass** der erste Differenzdrucksensor (281) in dem inneren Gasdurchgang (100) stromabwärts des verformbaren Reservoirs (27) angeordnet ist.

8. Gasabgabevorrichtung nach einem der vorhergehenden Ansprüche, **dadurch gekennzeichnet, dass** die Verarbeitungseinheit (51) einen Mikroprozessor umfasst, vorzugsweise einen Mikrocontroller.

9. Gasabgabevorrichtung nach einem der vorhergehenden Ansprüche, **dadurch gekennzeichnet, dass** der erste Differenzdrucksensor (281) einen ersten Abfühlanschluss bei atmosphärischem Druck und einen zweiten Abfühlanschluss, der in dem inneren Durchgang (100) angeordnet ist, umfasst.

10. Gasabgabevorrichtung nach einem der vorhergehenden Ansprüche, **dadurch gekennzeichnet, dass** sie des Weiteren ein (oder mehrere) Einwegeventilelement(e) (280) umfasst, das/die in dem inneren Gasdurchgang (100) stromabwärts des verformbaren Reservoirs (27) angeordnet ist/sind.

11. Gasabgabevorrichtung nach einem der vorhergehenden Ansprüche, **dadurch gekennzeichnet, dass** der erste Differenzdrucksensor (281) zwischen dem verformbaren Reservoir (27) und dem Einwegeventilelement (280) angeordnet ist.

12. Gasabgabevorrichtung nach einem der vorhergehenden Ansprüche, **dadurch gekennzeichnet, dass** sie des Weiteren einen zweiten Differenzdrucksensor (29) umfasst, der so ausgestaltet ist, dass er den Druckabfall misst, der durch das Einwegeventil (280) generiert wird.

13. Gasabgabevorrichtung nach einem der vorhergehenden Ansprüche, **dadurch gekennzeichnet, dass** der zweite Differenzdrucksensor (29) in einer Umgehungsleitung (290), die fließtechnisch mit dem inneren Gasdurchgang (100) verbunden ist, an stromaufwärtigen und stromabwärtigen Positionen (29a, 29b) des Einwegeventils (280) angeordnet ist.

14. Gasabgabevorrichtung nach einem der vorhergehenden Ansprüche, **dadurch gekennzeichnet, dass** der zweite Differenzdrucksensor (29) Drucksignale an die Verarbeitungseinheit (51) abgibt.

15. Gasabgabeanordnung (1, 3, 10), umfassend:

    - eine Gasabgabevorrichtung (1), umfassend einen inneren Gasdurchgang (100) nach einem der vorhergehenden Ansprüche,
    - eine Gasquelle (3), wie einen Gaszylinder (30), der mit einem Ventil (31) ausgestattet ist, in Fluidkommunikation mit dem inneren Gasdurchgang (100) der Gasabgabevorrichtung (1) zum Bereitstellen eines Atemgases an die Gasabgabevorrichtung (1), und
    - eine Beatmungsschnittstelle (10), wie eine Beatmungsmaske, in Fluidkommunikation mit dem inneren Gasdurchgang (100) der Gasabgabevorrichtung (1) zum Empfangen des durch den inneren Gasdurchlass (100) bereitgestellten Atemgases.

**Revendications**

1. Dispositif d'administration de gaz (1) comprenant un passage de gaz interne (100) en communication fluidique avec un réservoir déformable (27), et une unité de traitement (51), **caractérisé en ce qu'**il comprend en outre :

    - un premier capteur de pression différentielle (281) coopérant avec l'unité de traitement (51) pour déterminer une pression (P) dans le réservoir déformable (27), et
    - une vanne proportionnelle (22) agencée sur le passage de gaz interne (100) pour commander le débit de gaz dans ledit passage de gaz interne (100), ladite vanne proportionnelle (22) étant agencée en amont du réservoir déformable (27),

l'unité de traitement (51) commandant la vanne proportionnelle (22) pour ajuster le débit de gaz passant par ladite vanne proportionnelle (22) sur la base de ladite pression (P) dans le réservoir déformable (27).

2. Dispositif d'administration de gaz selon la revendication 1, **caractérisé en ce que** le premier capteur de pression différentielle (281) est configuré ou commandé pour effectuer des mesures de la pression (P) à des intervalles de temps donnés, de préférence toutes les 20 msec ou moins, plus préférentiellement toutes les 5 msec ou moins.

3. Dispositif d'administration de gaz selon l'une quelconque des revendications précédentes, **caractérisé en ce que** l'unité de traitement (51) est configurée pour traiter au moins un signal de mesure de pression délivré par le premier capteur de pression différentielle (281) et calculer la pression (P) à l'aide d'au moins un signal de mesure de pression traité.

4. Dispositif d'administration de gaz selon l'une quelconque des revendications précédentes, **caractérisé en ce que** l'unité de traitement (51) est configurée pour commander la vanne proportionnelle (22) pour régler ou modifier le débit de gaz traversant ladite vanne proportionnelle (22), proportionnellement à la pression (P).

5. Dispositif d'administration de gaz selon l'une quelconque des revendications précédentes, **caractérisé en ce que** le premier capteur de pression différentielle (281) est agencé à proximité du réservoir déformable (27).

6. Dispositif d'administration de gaz selon l'une quelconque des revendications précédentes, **caractérisé en ce que** l'unité de traitement (51) commande la vanne proportionnelle (22) pour augmenter ou pour diminuer le débit de gaz traversant la vanne proportionnelle (22) sur la base de la pression (P).

7. Dispositif d'administration de gaz selon l'une quelconque des revendications précédentes, **caractérisé en ce que** le premier capteur de pression différentielle (281) est agencé dans le passage de gaz interne (100) en aval du réservoir déformable (27).

8. Dispositif d'administration de gaz selon l'une quelconque des revendications précédentes, **caractérisé en ce que** l'unité de traitement (51) comprend un microprocesseur, de préférence un microcontrôleur.

9. Dispositif d'administration de gaz selon l'une quelconque des revendications précédentes, **caractérisé en ce que** le premier capteur de pression différentielle (281) comprend un premier port de détection à la pression atmosphérique et un deuxième port de détection agencé dans le passage interne (100).

10. Dispositif d'administration de gaz selon l'une quelconque des revendications précédentes, **caractérisé en ce qu'**il comprend en outre un (ou plusieurs) éléments de vanne unidirectionnelle (280) agencés dans le passage de gaz interne (100) en aval du réservoir déformable (27).

11. Dispositif d'administration de gaz selon l'une quelconque des revendications précédentes, **caractérisé en ce que** le premier capteur de pression différentielle (281) est agencé entre le réservoir déformable (27) et l'élément de vanne unidirectionnelle (280).

12. Dispositif d'administration de gaz selon l'une quelconque des revendications précédentes, **caractérisé en ce qu'**il comprend en outre un second capteur de pression différentielle (29) agencé de manière à mesurer la perte de charge générée par ladite vanne unidirectionnelle (280).

13. Dispositif d'administration de gaz selon l'une quelconque des revendications précédentes, **caractérisé en ce que** le second capteur de pression différentielle (29) est agencé dans un conduit de dérivation (290) relié fluidiquement au passage de gaz interne (100), à des emplacements en amont et en aval (29a, 29b) de la vanne unidirectionnelle (280).

14. Dispositif d'administration de gaz selon l'une quelconque des revendications précédentes, **caractérisé en ce que** le second capteur de pression différentielle (29) délivre des signaux de pression à l'unité de traitement (51).

15. Ensemble d'administration de gaz (1, 3, 10) comprenant :

- un dispositif d'administration de gaz (1) comprenant un passage de gaz interne (100) selon l'une quelconque des revendications précédentes,
- une source de gaz (3), telle qu'une bouteille de gaz (30) équipée d'une vanne (31), en communication fluidique avec le passage de gaz interne (100) du dispositif d'administration de gaz (1) pour fournir un gaz respiratoire au dispositif d'administration de gaz (1), et
- une interface respiratoire (10), telle qu'un masque respiratoire, en communication fluidique avec le passage de gaz interne (100) du dispositif d'administration de gaz (1) pour recevoir le gaz respiratoire fourni par ledit passage de gaz interne (100).

FIG.1

EP 3 701 992 B1

FIG.2

FIG.3

FIG.4

FIG. 5

EP 3 701 992 B1

FIG. 6

EP 3 701 992 B1

EP 3 701 992 B1

FIG. 7

FIG. 8

FIG. 9

**EP 3 701 992 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016063168 A